# EUROPEAN PATENT APPLICATION

(11) **EP 4 006 143 A1**
(43) Date of publication of application: **01.06.2022**
(21) Application number: 20844664.1
(22) Date of filing: 23.07.2020
(51) Int. Cl.: C12N 5/071, C12M 3/00, C09D 11/04, B29C 64/209, B33Y 80/00

(54) **LIVER ORGANOID AND PREPARATION METHOD THEREFOR**

(30) Priority: 23.07.2019 KR 20190089027
(71) Applicant: T&R Biofab Co., Ltd., Gyeonggi-do 15073 (KR)
(72) Inventor: JIN, Song Wan, Seongnam-si, Gyeonggi-do 13487 (KR); AHN, Geun Seon, Seongnam-si, Gyeonggi-do 13487 (KR); KANG, Dong Gu, Seongnam-si, Gyeonggi-do 13487 (KR)
(74) Representative: Caspary, Karsten
(86) International application number: PCT/KR2020/009722
(87) International publication number: WO 2021/015572

(57) **Abstract**

The present disclosure relates to a liver organoid, more specifically, to a liver organoid in which a liver lobule-type structure can be maintained for a long time and a preparation method using the same. The liver organoid of the present disclosure comprises: a tubular outer cavity the inside of which is divided into a plurality of compartments; a cell aggregate loaded into the compartments; and an inner cavity located at the core of the outer cavity.

## Description

### Technical Field

The present disclosure relates to a liver organoid and, more particularly, to a liver organoid in which a liver lobule-type structure can be maintained for a long time and a method for preparing the same.

### Background Art

Development of new drugs requires a lot of time, effort, and considerable cost. However, when it succeeds, high added value can be created. In order for a drug to be effective, the drug must reach a target site to the extent that it has a sufficient concentration to exhibit its effect. However, when the drug is metabolized in the liver, the drug loses its activity.

The drug-induced hepatotoxicity mechanism is closely related to liver metabolic activation. Drug-induced hepatotoxicity is the cause of stopping the development of a new drug in the preclinical stage and the cessation of clinical trials in the clinical stage. In addition, when drug-induced hepatotoxicity is found even after the drug is marketed, the drug is withdrawn from the market. Therefore, the development of three-dimensional liver models for toxicity and drug metabolism studies is important.

The liver is one of the largest organs of our body and functions to produce thousands of substances and enzymes necessary in our body, including bile, and detoxify various toxic substances.

The liver structure is very complex, and in general, the liver has a unit structure of liver lobules. These liver lobules consist of three interlobular veins that send out central vein and capillaries at the central location. The sinusoidal capillaries from the interlobular vein are surrounded by hepatocytes, and canalicular ducts are formed between the hepatocytes. Between the sinusoidal capillaries and the hepatocytes, there is a narrow duct called the space of Disse, and the hepatic stellate cells are located here. Due to such structural complexity, it is very difficult to fabricate a three-dimensional liver model similar to a human liver, and in particular, hepatocytes rapidly deteriorate in cell function during a two-dimensional culture.

Recently, research for developing a three-dimensional liver model using an organoid is being actively conducted. An organoid called a mini-organ is a structure prepared using progenitor cells or stem cells originating from a specific organ or tissue and refers to a three-dimensional structure having a structure and function similar to that of an actual human organ.

In a recent study, using adult stem cells extracted from the human liver, liver organoids having a structure and function similar to that of the human liver were successfully made in vitro. However, this method has disadvantages in that it is very difficult to extract the cells necessary for preparing liver organoids from the human body, and since the function of the cells is different for each donor, there is a difference between the prepared samples. In addition, cells cannot be arranged in the desired form because they are cultured through the self-assembly of cells.

In addition to organoid technology, a three-dimensional culture technology using a specific device such as a microfluidic device and a three-dimensional patterning chip using lithography is also used, but the cost is high, and the experimental procedure is very complicated. Although the spheroid technology allows a long-term culture by aggregating cells without bio-ink, there is a limitation in that it must be prepared only with a size of about 200 to 300 µm because a hypoxic state occurs in the center of the aggregated cells.

Bioprinting technology is currently the most studied technology, together with organoids. In order to mimic the structure of a human liver, each bio-ink containing various cells is ejected and stacked at a desired location using multiple heads to fabricate a liver analog with a size of several millimeters. However, the conventional bioprinting technology has a limitation in printing resolution, and in the case of preparing a large structure, oxygen reduction occurs due to stacking.

### Disclosure

### Technical Problem

An objective of the present disclosure is to provide a liver organoid capable of preventing a hypoxic state by forming a liver lobule-type structure so as to enable long-term cultivation and at the same time maintain a liver lobule-type structure for a long time and a preparing method using the same. The present disclosure can provide a liver organoid and a preparing method available for both non-clinical and clinical trials by maintaining a structure in liver lobule-type for a long time.

### Technical Solution

One embodiment of the present disclosure relates to a liver organoid, and a liver organoid includes: a tubular outer cavity 100 in which an inside is divided into a plurality of compartments 110; a cell aggregate 200 filling each of the plurality of the compartments 110; and an inner cavity 300 located at the center of the outer cavity 100.

The inner cavity 300 is preferably a tubular structure with an empty interior.

In addition, the outer cavity 100 may include vascular endothelial cells and a hydrogel, and the cell aggregate 200 may include hepatocytes and a hydrogel.

The hydrogel may include at least one selected from the group consisting of alginate, fibrin gel, carboxymethyl cellulose, heparan sulfate, hyaluronic acid, collagen, dextran, agarose, gelatin, laminin, Pluronic, bio-ink decellularized in specific tissues, Matrigel, hydroxyapatite, hyaluronic acid, and polyethylene glycol, or a mixed hydrogel in which at least two or three or more kinds of hydrogels are mixed may be used.

On the other hand, there is a method for preparing a liver organoid as another embodiment of the present disclosure. A method for preparing a liver organoid includes: a receiving part preparing step of preparing a bio-ink receiving part 2 provided with the partition member 1 that provides a partitioned space to have a cross-sectional pattern of the liver lobule; a bio-ink supplying step of supplying different bio-ink to each partitioned space of the bio-ink receiving part 2; and a discharging step of discharging different bio-ink accommodated in a partitioned space to a single nozzle 3 by applying a physical force to the bio-ink receiving part 2.

The partition member 1 preferably includes: a third hollow part 30 formed at the center of the partition member 1 having a ring-shaped cross-section; a plurality of second hollow parts 20 formed outside the third hollow part 30 having a fan-shaped cross-section; and a first hollow part 10 formed in a structure surrounding the second hollow part 20 and the third hollow part 30.

In the bio-ink supplying step, it is preferable that a first bio-ink 11 is supplied to a first hollow part 10, a second bio-ink 21 is supplied to a second hollow part 20, and a third bio-ink 31 is supplied to a hollow part 30. It is preferable that the first bio-ink 11 includes vascular endothelial cells and a hydrogel, the second bio-ink 21 includes hepatocyte and a hydrogel, and the third bio-ink 31 includes a hydrogel.

The hydrogel may include any one or more selected from the group consisting of alginate, fibrin gel, carboxymethyl cellulose, heparan sulfate, hyaluronic acid, collagen, dextran, agarose, gelatin, laminin, Pluronic, bio-ink decellularized in specific tissues, Matrigel, hydroxyapatite, hyaluronic acid, and polyethylene glycol.

It is preferable that the pressure applied to a bio-ink receiving part 2 in the discharging step is 0.1 to 500 kPa.

The discharging step may be performed simultaneously or sequentially with the bio-ink supplying step.

### Advantageous Effects

According to the present disclosure, the liver organoid is structurally stable because vascular endothelial cells surround the outer cavity and the inner cavity, and vascular endothelial cells are also connected between the outer cavity and the inner cavity. Even when a liver organoid is cultured for a long time, the shape may be maintained without structural change.

In addition, the liver organoid of the present disclosure may prevent cell death due to a hypoxic state by including an inner cavity that supplies oxygen, thereby enabling a long-term culture.

### Description of Drawings

FIG. 1 is a perspective view of a liver organoid according to an embodiment of the present disclosure;
FIG. 2 is a schematic diagram showing the preparing process of liver organoid according to another embodiment of the present disclosure; and
FIGS. 3 to 5 are results observed by culturing liver organoids according to the present disclosure.

### Best Mode

Hereinafter, prior to being described in detail through a preferred embodiment of the present disclosure, the terms or words used in the present specification and claims should not be construed as being limited to conventional or dictionary meanings and should not be construed in the technical spirit of the present disclosure. It should be interpreted in accordance with the meaning and concept.

Throughout this specification, when a part "includes" a certain component, it means that other components may be further included rather than excluding other components unless otherwise stated.

Hereinafter, the liver organoid of the present disclosure and its preparation method will be described in more detail.

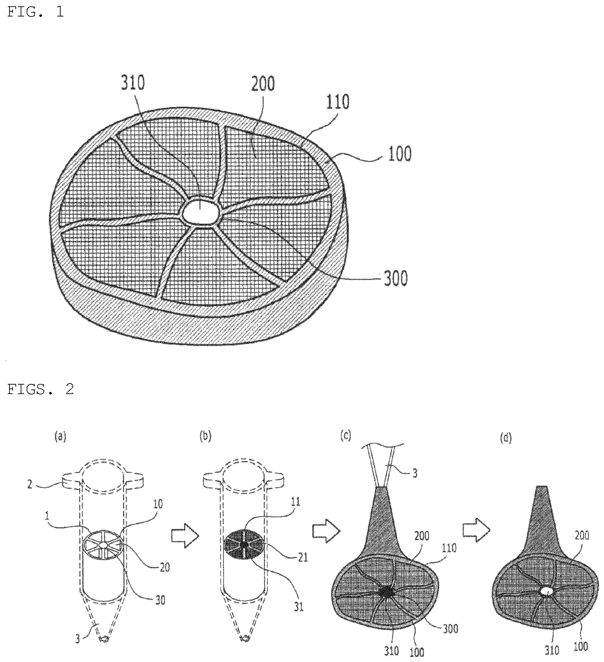

FIG. 1 is a perspective view of a liver organoid according to an embodiment of the present disclosure.

Referring to FIG. 1, a liver organoid according to an embodiment of the present disclosure includes: a tubular outer cavity 100 in which an inside is divided into a plurality of compartments 110; a cell aggregate 200 filling each of the plurality of the compartments 110; and a tubular inner cavity 300 located at the center of the outer cavity 100 having an empty inside. Therefore, a liver organoid has a structure similar to an actual liver lobule.

A through-hole 310 is formed in the center of the inner cavity 300.

The outer cavity 100 serves to maintain the overall structure of the liver organoid, as shown in FIG. 1, may be formed in a tubular structure in which the inside is divided into a plurality of compartments 110.

Vascular endothelial cells and a hydrogel are included in the outer circumferential surface of the through-holes 310 formed between the outer cavity 100 and the plurality of compartments 110 and at the center of the inner cavity 300.

These vascular endothelial cells are located on the outer surface of the outer cavity 100, which is the surface where the liver organoid is in direct contact with the fluid, and become vascularized, as well as vascular endothelial cells prevent hepatocytes from escaping from the cell aggregate 200, thereby maintaining the structure of the liver organoid of the present disclosure for a long time.

In addition, vascular endothelial cells are also vascularized in the outer circumferential surface of the through-holes 310 formed between the plurality of compartments 110 and in the center of the inner cavity 300.

The hydrogel may include any one or more selected from the group consisting of alginate, fibrin gel, carboxymethyl cellulose, heparan sulfate, hyaluronic acid, collagen, dextran, agarose, gelatin, laminin, Pluronic, bio-ink decellularized in specific tissues, Matrigel, hydroxyapatite, hyaluronic acid, and polyethylene glycol. Since such a hydrogel has high water content, excellent biocompatibility, and excellent mechanical properties, it is very suitable for application to an organoid having a structure including cells, such as the liver organoid of the present disclosure.

A cell aggregate 200 filling each of the plurality of the compartments 110 of the outer cavity 100 includes hepatocytes and a hydrogel. A hydrogel induces aggregation between hepatocytes by providing pores to which cells cannot adhere.

In addition, the cell aggregate 200 may further include cell growth factors, such as cytokines, in addition to hepatocytes and a hydrogel. A Cell growth factor is a substance that affects cell growth and differentiation and refers to proteins, polypeptides, or complexes thereof containing cytokines. As such a cell growth factor may include insulin, insulin-like growth factor (IGF), nerve growth factor (NGF), vascular endothelial growth factor (VEGF), keratinocyte growth factor (KGF), fibroblast growth factor (FGF), including basic FGF (bFGF), platelet-derived growth factor (PDGF) including PDGF-AA and PDGF-AB, bone morphogenetic protein (BMP) including BMP-2 and BMP-7, hepatocyte growth factor (HGF), transforming growth factor-alpha (TGF-α), transforming growth factor-beta (TGF-β,) including TGFβ-1 and TGFβ-3, epidermal growth factor (EGF), granulocyte-macrophage colony-stimulating factor (GM-CSF), granulocyte colony-stimulating factor (GCSF), interleukin-6 (IL-6), IL-8, and the like.

The inner cavity 300 is located in the central region of the outer cavity 100 constituting the liver organoid of the present disclosure and has a hollow tubular structure. The inner cavity 300 has a through-hole 310 formed in the center, through which oxygen can be supplied to the liver organoid. Supplying oxygen to hepatocytes included in the cell aggregate 200 through the inner cavity 300 make it possible to prevent hepatocytes from becoming hypoxic and dying. Accordingly, the liver organoid of the present disclosure can be cultured for a long time, and the structure is stably maintained even after a long-term culture.

In order to form the through-hole 310 in the central region of the inner cavity 300, the central region of the inner cavity 300 is preferably formed of a cell-free hydrogel, in particular, is preferably formed of a cell-free hydrogel that can be easily removed after discharge.

For example, collagen mixed with cells (gelled at a temperature of 37 degrees) may be added to the partition member, and alginate (gelled by calcium ions) in which cells are not mixed may be added to the central region of the inner cavity. When the discharged organoid is gelled at 37 degrees, the discharged organoid is gelled (hardened) in the desired shape, and alginate without calcium ions remains like water. When this is washed with the cell culture solution, the partition member is gelled and remains unwashed, and the alginate in the center of the lumen is washed out to form a lumen corresponding to the through-hole. That is, by not gelating only the hydrogel in the central region of the inner cavity to form a penetration hole or lumen, the hydrogel can be induced to be washed with the cell culture liquid and diffused and disappear.

FIGS. 2 is a schematic diagram schematically showing a process for producing liver organoids according to an embodiment of the present disclosure.

Referring to FIGS. 2, a method for producing liver organoid of the present disclosure is a method using bio-ink discharging. A method for preparing a liver organoid may include: a receiving part preparing step of preparing the bio-ink receiving part 2 provided with the partition member 1 that provides a partitioned space to have a cross-sectional pattern of the liver lobule (FIG. 2(a)); a bio-ink supplying step of supplying different bio-ink to each partitioned space of the bio-ink receiving part 2 (FIG. 2(b)); and a discharging step of discharging different bio-ink accommodated in a partitioned space to a single nozzle 3 by applying a physical force to the bio-ink receiving part 2 (FIG. 2(c)).

A partition member 1 in a receiving part preparing step is preferably formed to have a cross-sectional pattern corresponding to the cross-sectional pattern of the liver organoid of the present disclosure described above. Specifically, as shown in FIG. 2(a), the structure of the partition member 1 may include: a third hollow part 30 formed at a center of a partition member 1 having a ring-shaped cross-section; a plurality of second hollow parts 20 formed outside the third hollow part 30 having a fan-shaped cross-section; and a first hollow part 10 formed in a structure surrounding the second hollow part 20 and the third hollow part 30.

This first hollow part 10 corresponds to the outer cavity 100 of the liver organoid described above, the second hollow part 20 corresponds to the cell aggregate 200, and the third hollow part 30 corresponds to the through-hole 310 located in the center of the inner cavity 300.

A partition member 1 may be produced by the FDM printing method using a resin such as acrylonitrile butadiene styrene (ABS), polycaprolactone (PCL), acrylonitrile-styrene-acrylate (ASA), styrene-acrylonitrile copolymer (SAN), polystyrene (PS), PPSF /polyphenylsulfone (PPSU), polyetherimide, polylactic acid (PLA), poly-d-lysine (PDL), etc., or may be produced by a machining method using non-ferrous or non-ferrous alloy.

A bio-ink supplying step is a step of supplying different bio-inks to each space of a bio-ink receiving unit 2 partitioned by a partition member 1. Specifically, as shown in FIG. 2(b), a first bio-ink 11 may be supplied to a first hollow part 10, and a second bio-ink 21 may be supplied to a second hollow part 20, and a third bio-ink 31 may be supplied to a third hollow part 30.

A first bio-ink 11 is for forming a blood vessel between the above-described outer cavity 100, the inner cavity 300, and the compartment 110, and preferably includes vascular endothelial cells and a hydrogel.

A second bio-ink 21 is for forming the cell aggregate 200 in the compartment 110 and may include hepatocytes and a hydrogel.

As the hepatocytes, for example, embryo-derived liver stem cells, induced pluripotent liver stem cells, adult liver stem cells, primary stem cells, immortal cell lines, etc., may be used.

In addition, a second bio-ink 21 may further include a cell growth factor, which is a material that affects the growth and differentiation of cells, in addition to hepatocytes and a hydrogel. As such cell growth factors may include insulin, insulin-like growth factor (IGF), nerve growth factor (NGF), vascular endothelial growth factor (VEGF), keratinocyte growth factor (KGF), fibroblast growth factor (FGF), including basic FGF (bFGF), platelet-derived growth factor (PDGF) including PDGF-AA and PDGF-AB, bone morphogenetic protein (BMP) including BMP-2 and BMP-7, and hepatocyte growth factor (HGF), transforming growth factor-alpha (TGF-α), transforming growth factor-beta (TGF-β) including TGFβ-1 and TGFβ-3, epidermal growth factor (EGF), granulocyte-macrophage colony-stimulating factor ( GM-CSF), granulocyte colony-stimulating factor (GCSF), interleukin-6 (IL-6), IL-8, and the like.

In addition, a third bio-ink 31 is for forming the through-hole 310 located in the center of the above-described inner cavity 300, and a third bio-ink is preferable to include a hydrogel.

The hydrogel may include any one or more selected from the group consisting of alginate, fibrin gel, carboxymethyl cellulose, heparan sulfate, hyaluronic acid, collagen, dextran, agarose, gelatin, laminin, Pluronic, bio-ink decellularized in specific tissues, Matrigel, hydroxyapatite, hyaluronic acid, and polyethylene glycol.

The first hollow part and the third hollow part can accommodate different hydrogels having different gelation conditions. For example, alginate, which is gelled by calcium, is placed in the first hollow part. When gelatin, which is gelled at less than about 30 degrees and exists as a fluid at more than 30 degrees, is put into the third hollow and then discharged into a 37 degrees cell culture solution containing calcium ions, alginate is immediately gelled, and gelatin is not gelled.

Therefore, gelatin diffuses into the cell culture solution in a fluid state and melts away, thereby forming a through-hole 310 located in the center of the inner cavity, and only alginate is maintained so that a unique liver organoid according to the present disclosure can be prepared.

As shown in FIG. 2(c), the discharging step is a step of discharging different bio-ink accommodated in the partitioned space to a single nozzle 3 by applying pressure to the bio-ink receiving part 2. At this time, it is preferable that the same pressure is applied to each of the different bio-inks.

The pressure applied to the receiving part 2 is preferably in the range of about 0.1 to 500 kPa. If the pressure is too strong and exceeds 500 kPa, the load applied to the nozzle 3 may become large, and damage may occur. In addition, when the pressure is less than 0.1 kPa, it is not smoothly discharged from the nozzle 3 due to the resistance due to the viscosity of the bio-ink.

The diameter of the nozzle 3 is preferably about 0.1 to 100 mm. When the diameter of the nozzle 3 is less than 0.1 mm, the discharge pressure may be increased to cause damage to cells, and when the diameter of the nozzle exceeds 100 mm, there is a problem in that the precision is lowered as well as the consumption of the substance is increased too much.

This discharging step may be performed simultaneously with the bio-ink supplying step or may be performed sequentially.

The liver organoids formed by being discharged in the discharging step may be used as they are, and if necessary, cut to a predetermined thickness and used or continuously laminated in a specific pattern to form the desired shape.

For example, the liver organoid may be used by pulling longitudinal direction in the form of a fiber, or it can be cut and used and can be continuously laminated in the desired shape, preferably discharged with a nozzle having a diameter of 1 mm. This is because the human liver lobule is about 1 mm in size, and if the structure discharged in the form of long fiber is cut back to 1 mm, a structure very similar to that of the liver lobule can be obtained. In addition, when it is made into a large mass by continuous discharge and lamination processing without cutting it, it is also possible to make lobes of the liver in which liver lobules are arranged in various structures.

Hereinafter, an embodiment of the present disclosure will be described. However, the scope of the present disclosure is not limited to the following preferred embodiments and those of ordinary skilled in the art to which the present disclosure pertains can take various modified forms of the contents described herein within the scope of the present disclosure.

### [Example 1] Preparation of a first bio-ink

A first bio-ink was prepared by adding vascular endothelial cells to an aqueous collagen solution that was neutralized to pH 6.0 to 8.0 and stirred.

### [Example 2] Preparation of a second bio-ink

A second bio-ink was prepared by adding human adult liver stem cells to an aqueous collagen solution that was neutralized to pH 6.0 to 8.0 and stirred.

### [Example 3] Preparation of a third bio-ink

A third bio-ink was prepared by adding alginate to distilled water and stirring at room temperature for 12 hours.

### [Example]

In order to prepare the liver organoid of the present disclosure, a partition member has a first hollow part, a second hollow part, and a third hollow part, as shown in FIG. 2(a) was prepared using polylactic acid (PLA) as a substance discharge method.

After inserting the partition member prepared in the method mentioned above into the receiving part of the 3D printer syringe, the first bio-ink prepared in the preparation mentioned above Example was supplied to the first hollow part, and the second bio-ink to the second hollow part was supplied, and the third bio-ink was supplied to the third hollow part. Thereafter, a pressure of about 10 kPa was applied using pneumatic pressure to discharge the bio-ink from a single nozzle (diameter: 1 mm) to prepare a liver organoid.

### [Comparative Example]

In order to prove the effectiveness of the liver organoid of the present disclosure, two comparison groups (Mix (W/O lumen) vs Mix (W lumen)) were set, and an experiment was conducted. First, hepatocytes (HepG2, hereinafter the same) and vascular endothelial cells (EA.hy926, hereinafter the same) were mixed in a ratio of about 7:3 and mixed with bio-ink, and then the mixed bio-ink was supplied to a syringe without a partition member. After that, a pressure of 10 kPa was applied using pneumatic pressure, and the bio-ink was discharged from a single nozzle (diameter: 1 mm) to prepare liver organoid (Mix W/O lumen). Then, hepatocytes and vascular endothelial cells were mixed in a ratio of about 7:3 and mixed with bio-ink, and liver organoid in which the two cells were mixed was prepared in the same way as liver organoid (Mix W lumen).

As a result, the mixed model, which did not make the lumen, had a hypoxic state in the middle of the gel, and the structure was severely deformed as the cells survived only outside. In the mixed model in which the lumen was made, hepatocytes, not vascular endothelial cells, grew together in the lumen. The lumen was vascularized, and the structure could not be maintained. As the two cells grew arbitrarily, the lumen was filled, and eventually, the cell survival rate decreased.

### [Experimental Example 1]

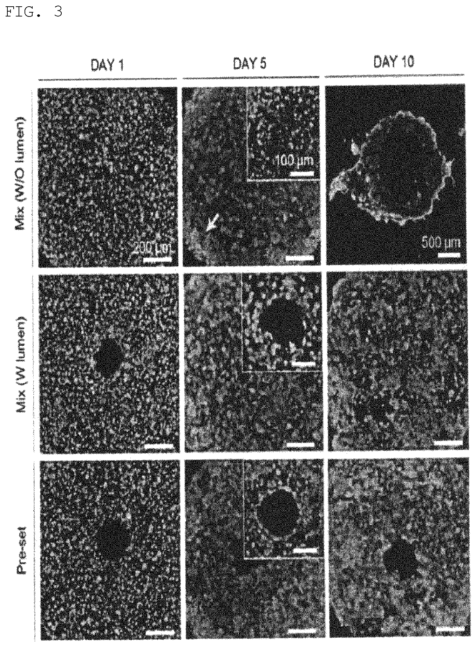

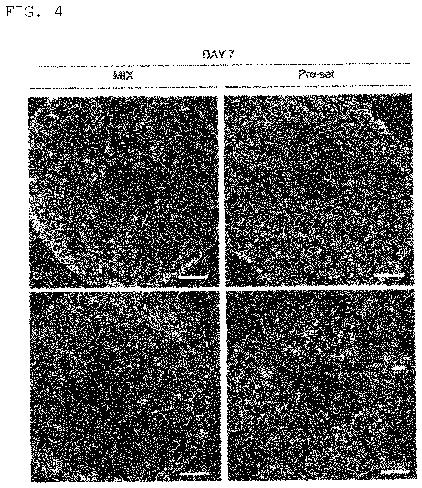

After culturing the liver organoid of Examples and Comparative Examples for 10 days, they were observed under a microscope and shown in FIGS. 3 and 4 (MIX in FIG. 4 is the same as Mix (W lumen) in FIG. 3, and Pre-set of FIG. 4 is the same sample as the Pre-set of FIG. 3).

Mix (W/O lumen) of FIG. 3 is the result of mixing hepatocytes and vascular endothelial cells in a hydrogel at the same time and discharging them through a 1 mm diameter nozzle without any pattern. Mix (W lumen) is the result of simultaneously mixing hepatocytes and vascular endothelial cells with a hydrogel and discharging them in the same manner as the liver organoid preparing method. Pre-set is the result of producing liver organoids in a predetermined pattern, allowing heterogeneous distinction between hepatocytes and vascular endothelial cells. In particular, in the case of the Pre-set, it can be seen that the vascular endothelial cells maintain their structure well by connecting the outer cavity to the inner cavity and between them.

### [Experimental Example 2]

In order to confirm that the structure of the liver organoid of Example is maintained even when cultured for a long period of time, an experiment was performed in the same manner as in Example.

As a first embodiment, collagen in the blood vessel part, collagen in the hepatocyte part, and alginate in the inner cavity were respectively used, and the mixed bio-ink was discharged in the form of long fibers in a cell culture solution at a temperature of 37 degrees at a pressure of 10 kPa through a 1 mm diameter nozzle. Afterward, it was transferred to an incubator.

Since collagen is gradually gelled at a temperature of 37 degrees, when discharged in a culture solution of 37 degrees, it is gelled and exists in the form of fibers in the solution. When gelation is complete, the alginate located in the inner hole is diffused and dissolved in the media solution.

As a second embodiment, an experiment was performed using collagen and alginate in the blood vessel part, collagen and alginate in the hepatocyte part, and alginate in the inner cavity.

Here, in the case of a mixture of collagen and alginate, a mixture was prepared in which 3% by weight of collagen and 3% by weight of alginate neutralized to pH 7.0 were mixed in a volume ratio of 9:1, and then the mixture was mixed with each cell and used, only 3% by weight of alginate was used alone for the inner cavity.

Similar to the above-mentioned first embodiment, after discharge in the form of long fibers in the cell culture medium containing calcium at a pressure of 10 kPa through a 1 mm diameter nozzle, it was allowed to stand for about 5 minutes. In the case of alginate, it gels as soon as when it meets calcium ions. During the process of standing for about 5 minutes, only the alginate component in the alginate and collagen mixture on the outer surface is gelled, and the alginate on the inside does not gel.

Thereafter, the cell culture medium containing calcium was washed with phosphate-buffered saline (PBS), and the calcium-free cell culture medium was put in and cultured in an incubator. After the collagen is sequentially gelled in this way, the alginate located in the inner cavity diffuses into the cell culture medium and melts away.

As a third embodiment, alginate was used in the blood vessel part, alginate in the hepatocyte part, and gelatin in the inner cavity. Similar to the first and second embodiments discussed above, after discharge in the form of long fibers in the cell culture medium containing calcium at a pressure of 10 kPa through a 1 mm diameter nozzle, it was allowed to stand for about 5 minutes.

After the stationary time, the cell culture medium containing calcium ions was washed with phosphate-buffered saline (PBS), and then the cell culture medium containing no calcium ions was put in and cultured in an incubator. At 37 degrees, since gelatin exists as a liquid, gelatin diffuses into the cell culture medium and melts away.

All samples of Examples 1 to 3 were cultured in a cell culture apparatus at 37 degrees with Dulbecco's modified Eagle medium (DMEM), a cell culture medium containing 10% bovine serum and 1% antibiotics in a 24-well plate. The culture medium was newly replaced once a day, and calcium ions were supplied by adding calcium chloride (CaCl₂) to a PBS or cell culture medium at a concentration of 50 mM for gelation of alginate.

In order to distinguish and observe two different types of cells, each of the two cells were immuno-stained. After 7 days of culture, the sample is washed 3 times with PBS and fixed with 4% formaldehyde for 5 minutes. The fixed sample was washed 3 times again with PBS and then permeabilized for 10 minutes using 0.25% Triton-X 100.

The permeabilized sample was washed 3 times with PBS and blocked with 1% bovine serum albumin for 30 minutes, and the blocked sample was grounded with an albumin primary anti-body and cultured in an incubator of 37 degrees for about 1 hour. After that, each sample is washed 3 times, and the secondary anti-body with fluorescence inherent in albumin is grounded and cultured in an incubator at 37 degrees for 1 hour.

Through this process, hepatocytes are stained with albumin. Each sample was sequentially washed 3 times, and the sample was grounded with the CD31 primary antibody and incubated for 1 hour in an incubator at 37 degrees. Thereafter, the samples were washed 3 times, respectively, and the sample was grounded with the secondary antibody containing fluorescence that was grounded to albumin and incubated in an incubator at 37 degrees for 1 hour. As a result, vascular endothelial cells were stained with CD31, and the samples were observed by scanning the samples in the z-axis direction with a thickness of 10 µm using confocal microscopy.

In the same manner, MRP2 was also grounded on hepatocytes and observed with confocal laser scanning microscopy.

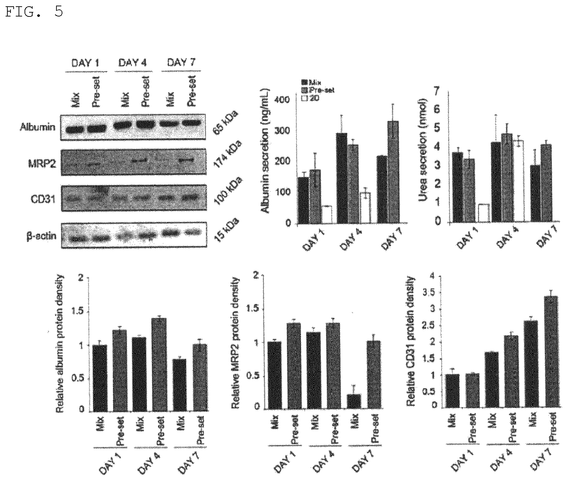

Albumin is a protein secreted by hepatocytes that are specifically stained by hepatocytes, MRP2 is a pump protein that discharges bile pigment from hepatocytes into the canalicular ducts and is specifically stained in hepatocytes, and CD31 is expressed and stained specifically in vascular endothelial cells with PECMA-1 (see FIG. 5).

Western blotting was performed to confirm the expression level of specific proteins (albumin, MRP2, CD31). First, a mixed protein of a sample (MIX W lumen, Pre-set) was extracted and normalized to the same concentration. After electrophoresis of the mixed protein on an agarose gel, the protein was transferred to a nylon membrane, after washing the protein-membrane with tris-buffered saline (TBS), blocking with 1% BSA for 1 hour, sequentially reacting with a primary antibody and a secondary antibody, and then measuring the protein expression level (band) using a light-emitting device.

In conclusion, it was confirmed that the protein expression and maintenance of liver organoid (Pre-set) was significantly higher than that of the mixed model (Mix W lumen), and the structural maintenance was also stable (see FIG. 4).

### Industrial Applicability

The liver organoid, according to the present disclosure, has a structure in the form of a liver lobule-type and can prevent a hypoxic state so that it can be cultured for a long period of time, while at the same time, the structure of the liver lobule-type is maintained for a long time. Therefore, industrial applicability exists.

## Claims

1. A liver organoid comprising:
a tubular outer cavity (100) having an inner space divided into a plurality of compartments (110);
a cell aggregate (200) filling each of the plurality of the compartments (110); and
an inner cavity (300) provided at the core of the outer cavity (100),
wherein a through-hole (310) having a tubular hollow structure that is empty is formed at the center of the inner cavity (300),
each of the outer cavity (100) and the inner cavity (300) comprises vascular endothelial cells, and
blood vessels are formed between an outer surface of the outer cavity (100) and each of the plurality of compartments (110) and on an outer circumferential surface of the through-hole (310).

2. The liver organoid of claim 1, wherein each of the outer cavity (100) and the inner cavity (300) further comprises a hydrogel, and the cell aggregate (200) comprises hepatocytes and a hydrogel.

3. The liver organoid of claim 2, wherein the hydrogel comprises at least one selected from the group consisting of alginate, fibrin gel, carboxymethyl cellulose, heparan sulfate, hyaluronic acid, collagen, dextran, agarose, gelatin, laminin, Pluronic, bio-ink decellularized in specific tissues, Matrigel, hydroxyapatite, hyaluronic acid, and polyethylene glycol.

4. A method of preparing a liver organoid, the method comprising:
a receiving part preparing step of preparing a bio-ink receiving part (2) provided with a partition member (1) that provides a partitioned space to have a cross-sectional pattern of a liver lobule;
a bio-ink supplying step of supplying a different bio-ink to each partitioned space of the bio-ink receiving part (2); and
a discharging step of discharging the bio-inks accommodated in the respective partitioned spaces through a single nozzle (3) by applying a physical force to the bio-ink receiving part (2),
wherein the partition member (1) preferably comprises a third hollow part (30) formed at the center of the partition member (1) having a ring-shaped cross-section, a plurality of second hollow parts (20) formed outside the third hollow part (30) and having a fan-shaped cross-section, and a first hollow part (10) formed to surround the second hollow part (20) and the third hollow part (30), and
wherein the bio-ink supplying step is **characterized in that** a first bio-ink (11) is supplied to the first hollow part (10), a second bio-ink (21) is supplied to the second hollow part (20), and a third bio-ink (31) is supplied to the third hollow part (30).

5. The method of claim 4, wherein the first to third bio-inks are different from each other.

6. The method of claim 4, wherein the first bio-ink (11) comprises a hydrogel and vascular endothelial cells.

7. The method of claim 4, wherein the second bio-ink (21) comprises hepatocytes and a hydrogel.

8. The method of claim 4, wherein the third bio-ink (31) comprises a cell-free hydrogel.

9. The method of claim 6, wherein the hydrogel in each of the first to third bio-inks comprises at least one selected from the group consisting of alginate, fibrinogen, carboxymethyl cellulose, heparan sulfate, hyaluronic acid, collagen, and dextran.

10. The method of claim 4, wherein a pressure applied to the bio-ink receiving part (2) in the discharging step is 0.1 to 500 kPa.

11. The method of claim 4, wherein the discharging step and the bio-ink supplying step are simultaneously performed or sequentially performed in this order.

12. The method of claim 9, wherein the first bio-ink comprises collagen and vascular endothelial cells, the second bio-ink comprises collagen and hepatocytes, and the third bio-ink comprises alginate.

13. The method of claim 9, wherein the first bio-ink comprises a mixture of collagen and alginate, and vascular endothelial cells,
the second bio-ink comprises a mixture of collagen and alginate, and hepatocytes, and
the third bio-ink comprises alginate.

14. The method of claim 9, wherein the first bio-ink comprises alginate and vascular endothelial cells,
the second bio-ink comprises alginate and hepatocytes, and
the third bio-ink comprises gelatin.

15. The method of claim 13, wherein the mixture of collagen and alginate is a mixture in which 3% by weight of collagen neutralized to pH 7.0 and 3% by weight of alginate are mixed in a volume ratio of 9:1.
